# EUROPEAN PATENT APPLICATION

(11) **EP 4 190 322 A1**
(43) Date of publication of application: **07.06.2023**
(21) Application number: 21850238.3
(22) Date of filing: 29.07.2021
(51) Int. Cl.: A61K 31/18, A61K 31/136, A61K 31/137, A61K 9/08, A61K 9/20, A61K 9/48, A61K 9/72, A61P 25/18, A61P 25/22, A61P 25/24

(54) **COMPOUND FOR PREVENTING AND TREATING PSYCHIATRIC DISORDERS AND USE THEREOF**

(30) Priority: 30.07.2020 CN 202010754144
(71) Applicant: Shanghai Institute of Materia Medica, Chinese Academy of Sciences, Pudong, Shanghai 201203 (CN); Shaoxing Zeroin Biomedicines Co., Ltd., Shaoxing, Zhejiang 312366 (CN)
(72) Inventor: YANG, Huaiyu, Shaoxing, Zhejiang 312366 (CN); LI, Yang, Shanghai 201203 (CN); GUO, Fei, Shanghai 201203 (CN); JIANG, Hualiang, Shanghai 201203 (CN); ZHANG, Qiansen, Shaoxing, Zhejiang 312366 (CN)
(74) Representative: Mewburn Ellis LLP
(86) International application number: PCT/CN2021/109365
(87) International publication number: WO 2022/022651

(57) **Abstract**

Provided are a compound for preventing and treating psychiatric disorders and the use thereof. In particular, provided is the use of a compound of formula I or a pharmaceutically acceptable salt thereof in (i) the preparation of a pharmaceutical composition or a preparation for preventing and/or treating TREK-1 ion channel-related diseases, (ii) the preparation of a pharmaceutical composition or a preparation for preventing and/or treating psychiatric disorders and/or (iii) the preparation of a TREK-1 ion channel inhibitor.

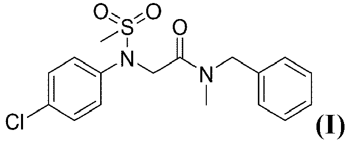

## Description

### TECHNICAL FIELD

The invention relates to the field of pharmaceutical chemistry, in particular, a compound for preventing and treating psychiatric disorders and use thereof.

### BACKGROUND

Depression is a common mood disorder that can lead to suicidal thoughts and behaviors in severe cases, and affects 16% of the population at some point in their lives. Depressive disorder is a serious mental disorder characterized by high prevalence, high relapse rate, high disability rate and high suicide rate. Depression also brings considerable economic burden while seriously affects the physical and mental health of patients. Depression is an extremely complex psychiatric disorder according to the comprehensive analysis of clinical diagnosis, pathological mechanism and treatment outcome.

Major depressive disorder (MDD) is a serious mental disorder with a low recovery rate and a high relapse rate, and its chronic incidence rate is about 20%. The annual cost of major depression is approximately $83 billion all over the world, which mainly includes indirect costs caused by reduced psychosocial function (especially workforce performance).

Refractory depression (TRD) comes from the treating goal for MDD. In general, the primary treating goal for MDD is to achieve a state of full recovery in which the affected individual no longer has clinically significant symptoms and/or functional impairment (e.g., at work, in personal and domestic life). In clinical practice, failure to achieve remission using two or more appropriate antidepressant treatments is considered as TRD.

Currently, antidepressant targets are primarily monoamine receptors and transporters, but these first-line clinical drugs have deficiencies such as slow onset, large individual differences, and ineffectiveness in 30% of patients.

In summary, there is an urgent need in this field to develop a novel antidepressant drug.

### SUMMARY OF THE INVENTION

The purpose of the present invention is to provide a novel drug for preventing or treating psychiatric disorders (such as depression).

In the first aspect of the present invention, provided is a use of a compound of formula I or a pharmaceutically acceptable salt thereof in (i) the preparation of a pharmaceutical composition or a preparation for preventing and/or treating a TREK-1 ion channel-related disease, (ii) the preparation of a pharmaceutical composition or a preparation for preventing and/or treating a psychiatric disorder and/or (iii) the preparation of a TREK-1 ion channel inhibitor.

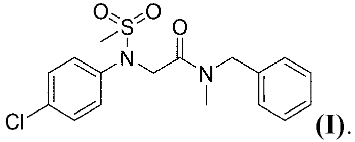

In another preferred embodiment, the compound of formula I is compound CLA096, i.e.
N-benzyl-2-(N-(4-chlorophenyl)methylsulfonylamino)-N-methylacetamide.

In another preferred embodiment, the TREK-1 ion channel-related disease refers to diseases that can be treated, prevented or improved by inhibiting the TREK-1 ion channel.

In another preferred embodiment, the TREK-1 ion channel-related diseases include a psychiatric disorder.

In another preferred embodiment, the psychiatric disorder includes depression, anxiety disorder, schizophrenia, bipolar disorder, or a combination thereof.

In another preferred embodiment, the TREK-1 ion channel-related disease includes depression, anxiety disorder, schizophrenia, bipolar disorder, or a combination thereof.

In another preferred embodiment, the depression includes major depressive disorder and/or refractory depression.

In another preferred embodiment, the preventing and/or treating TREK-1 ion channel-related disease includes depression, anxiety disorder, schizophrenia, bipolar disorder, or a combination thereof.

In another preferred embodiment, the preventing and/or treating a psychiatric disorder includes depression, anxiety disorder, schizophrenia, bipolar disorder, or a combination thereof.

In another preferred embodiment, the pharmaceutical composition further includes one or more additional active ingredient selected from the group consisting of
(i) a TREK-1 ion channel inhibitor;
(ii) a drug for preventing or treating a psychiatric disorder.

In another preferred embodiment, the additional active ingredient is not the compound of formula I and the pharmaceutically acceptable salt thereof.

In another preferred embodiment, the drug for preventing or treating a psychiatric disorder includes an active ingredient for treating depression or antidepressants.

In another preferred embodiment, the pharmaceutical composition further contains other active ingredients for treating depression.

In another preferred embodiment, the administration dosage of the compound of formula I is 0.1-50 mg/kg body weight, preferably 1-5 mg/kg body weight.

In another preferred embodiment, there is no adverse reaction at the administration dosage of the compound of formula I of ≤50 mg/kg body weight.

In another preferred embodiment, the adverse reactions include significant change in body weight and death.

In another preferred embodiment, the pharmaceutical composition contains 0.001 to 99 wt% the compound of formula I or the pharmaceutically acceptable salt thereof, based on the total weight of the pharmaceutical composition.

In another preferred embodiment, the pharmaceutical composition contains 0.1 to 90 wt%, preferably 1 to 80 wt%, the compound of formula I or the pharmaceutically acceptable salt thereof, based on the total weight of the composition.

In another preferred embodiment, the dosage form of the pharmaceutical composition includes an oral dosage form, an injection dosage form, and/or an inhalation dosage form.

In another preferred embodiment, the oral dosage form includes tablets, capsules, membranes, and/or granules.

In another preferred embodiment, the oral dosage form is a sustained-release dosage form or a non-sustained-release dosage form.

In the second aspect of the present invention, provided is a pharmaceutical composition, comprising a compound of Formula I or a pharmaceutically acceptable salt thereof.

In another preferred embodiment, the pharmaceutical composition further includes one or more additional active ingredients selected from the group consisting of
(i) a TREK-1 ion channel inhibitor;
(ii) a drug for preventing or treating a psychiatric disorder.

In another preferred embodiment, the additional active ingredient is not the compound of formula I and the pharmaceutically acceptable salt thereof.

In another preferred embodiment, the drug for preventing or treating a psychiatric disorder includes an active ingredient for treating depression or antidepressants.

In another preferred embodiment, the pharmaceutical composition further contains other active ingredients for treating depression or antidepressants.

In another preferred embodiment, the pharmaceutical composition further contains pharmaceutically acceptable carriers.

In another preferred embodiment, the total content of the compound of formula I or the pharmaceutically acceptable salt thereof is 0.001 to 99 wt%, preferably 0.1 to 90 wt%, more preferably 1 to 80 wt% of the pharmaceutical composition.

In another preferred embodiment, the pharmaceutical composition is used for preventing and/or treating a TREK-1 ion channel-related disease.

In another preferred embodiment, the pharmaceutical composition is used for preventing and/or treating a psychiatric disorder.

In another preferred embodiment, the TREK-1 ion channel-related disease includes a psychiatric disorder.

In another preferred embodiment, the psychiatric disorder includes depression (including major depressive disorder and/or refractory depression.), anxiety disorder, schizophrenia, bipolar disorder, or a combination thereof.

In another preferred embodiment, the pharmaceutical composition is used for preventing and/or treating major depressive disorder.

In another preferred embodiment, the pharmaceutical composition is used for preventing and/or treating refractory disorder.

In the third aspect of the present invention, provided is a TREK-1 ion channel inhibitor, comprising a compound of Formula I or a pharmaceutically acceptable salt thereof:

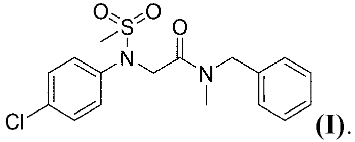

In the fourth aspect of the present invention, provided is a method for preventing and/or treating a TREK-1 ion channel-related disease comprising a step of administration of a compound of Formula I or a pharmaceutically acceptable salt thereof to a subject in need thereof; wherein the compound of formula I is as defined in the first aspect.

In another preferred embodiment, the TREK-1 ion channel-related disease is a psychiatric disorder.

In another preferred embodiment, the psychiatric disorder includes depression, anxiety disorder, schizophrenia, bipolar disorder, or a combination thereof.

In another preferred embodiment, the TREK-1 ion channel-related disease includes depression, anxiety disorder, schizophrenia, bipolar disorder, or a combination thereof.

In another preferred embodiment, the subject includes human people, or non-human mammals.

In another preferred embodiment, the non-human mammals include primates, or rodents (e.g., mice, rats, etc.).

In another preferred embodiment, the administration dosage of the administration is 1 to 20 mg/kg body weight, preferably 5 mg/kg body weight.

In another preferred embodiment, the frequency of the administration is 1 to 4 times per day, preferably 1 time a day.

In another preferred embodiment, the administration total daily dosage of the administration is 1 to 20 mg/kg body weight, preferably 5 mg/kg body weight.

In another preferred embodiment, the time period of the administration is from 2 weeks to 5 years, preferably from 2 months to 1 year.

In the fifth aspect of the present invention, provided is a method for preventing and/or treating a psychiatric disorder comprising a step of administration of a compound of Formula I or a pharmaceutically acceptable salt thereof to a subject in need thereof; wherein the compound of formula I is as defined in the first aspect.

In another preferred embodiment, the psychiatric disorder includes depression, anxiety disorder, schizophrenia, bipolar disorder, or a combination thereof.

In another preferred embodiment, the psychiatric disorder is depression.

In another preferred embodiment, the subject includes human people, or non-human mammals.

In another preferred embodiment, the non-human mammals include primates, or rodents (e.g., mice, rats, etc.).

In another preferred embodiment, the administration dosage of the administration is 1 to 20 mg/kg body weight, preferably 5 mg/kg body weight.

In another preferred embodiment, the frequency of the administration is 1 to 4 times per day, preferably 1 time a day.

In another preferred embodiment, the administration total daily dosage of the administration is 1 to 20 mg/kg body weight, preferably 5 mg/kg body weight.

In another preferred embodiment, the time period of the administration is from 2 weeks to 5 years, preferably from 2 months to 1 year.

In the sixth aspect of the present invention, provided is a kit, comprising:
(i) a drug or a preparation containing a compound of formula I or a pharmaceutically acceptable salt thereof as an active ingredient; and
(ii) an instructions.

In another preferred embodiment, the recording of the instructions notes that the indications of the kit include psychiatric disorder, preferably the indications include depression (including major depressive disorder and/or refractory depression.), anxiety disorder, schizophrenia, and/or bipolar disorder.

In another preferred embodiment, the recording of the instructions notes that the administration dosage of the drug or the preparation is 1 to 20 mg/kg body weight, preferably 5 mg/kg body weight, calculated by the active ingredient.

In another preferred embodiment, the administration frequency of the drug or the preparation is 1 to 4 times per day, preferably 1 time a day.

In another preferred embodiment, the administration time period of the drug or the preparation is from 2 weeks to 5 years, preferably from 2 months to 1 year.

In the seventh aspect of the invention, provided is a method for inhibiting the current of an intracellular TREK-1 ion channel, comprising a step of: contacting a cell expressing a TREK-1 ion channel protein with a compound of Formula I or a pharmaceutically acceptable salt thereof, thereby inhibiting the current of the intracellular TREK-1 ion channel.

In another preferred embodiment, the method is *in vitro* and non-therapeutic.

It should be understood that within the scope of the present invention, the above technical features of the present invention and the technical features specifically described in the following (e.g., embodiments) can be combined with each other, thereby forming a new or preferred technical solution. Due to space limitations, it will not be repeated herein.

### DESCRIPTION OF THE DRAWINGS

Figure 1 shows the TREK-1 ion channel action effect experiment of Example 2, wherein Figures A, B and C show the effect of CLA096 at 5 µM, 10 µM and 20 µM respectively, on the I-V curve (top), current amplitude (middle) and current inhibition rate (bottom) of the TREK-1 channel.
Figure 2 shows the effect of different doses of CLA096 on the immobility time of forced swimming in the forced swimming experiment in normal mice (Example 3) to evaluate the antidepressant-like effect of CLA096. ^{∗}*p*<0.05, ^{∗∗}*p*<0.01, ^{∗∗∗}*p*<0.001, compared with vehicle group.
Figure 3 shows the effect of different doses of CLA096 on the latency of feeding inhibition in novel environment in the novel environment feeding inhibition experiment in normal mice (Example 3) to evaluate the antidepressant-like effect of CLA096. ^{∗}*p*<0.05, ^{∗∗}*p*<0.01, compared with vehicle group.
Figure 4 shows the modeling process of the animal model of refractory depression in rats and the route map of administration (example 4).
Figure 5 shows the pharmacodynamic evaluation in the rat refractory depression model induced with adrenocorticotropic hormone (Example 4), wherein Figures A-C show the immobility, swimming and climbing states of the rats in the forced swimming experiment, respectively, and Figure D shows the total distance traveled by the rats in the open field experiment. ^{∗}*p*<0.05, compared with vehicle group.
Figure 6 shows the survival curve of mice in a 14-day subacute toxicity assay for CLA096 (Example 5).
Figure 7 shows the weight change of mice in the 14-day subacute toxicity experiment for CLA096, wherein the left panel shows the weight change of male mice and the right panel shows the weight change of female mice (Example 5).

### EMBODIMENTS FOR CARRYING OUT THE INVENTION

After extensive and in-depth research, the present inventors unexpectedly found that CLA096 has a significant inhibitory effect on the TREK-1 ion channel, and that the compound also has excellent *in vivo* antidepressant ability and low toxicity, therefore the compound is very suitable for the treatment or prevention of TREK-1 ion channel-related diseases (e.g., psychiatric disorders such as depression) or for the treatment or prevention of psychiatric disorders. The present invention is completed on this basis.

In particular, various antidepressant behavioral efficacy experiments of CLA096 were conducted herein. The experiments were conducted by intraperitoneal administration of CLA096 with different doses, and the compound CLA096 was found to have a significant antidepressant effect that was superior to that of the positive control fluoxetine, making it very suitable for application in the preparation of drugs for the treatment of depression.

### TERMS

Unless otherwise indicated, the abbreviations used herein have the conventional meaning as understood by those skilled in the art, for example, "Ms" refers to methylsulfonyl.

### Active Ingredients

As used herein, the terms "compound of the invention", "compound of Formula I", "CLA096" and "compound CLA096" are used interchangeably, referring to a compound shown in Formula I (whose name is N-benzyl-2-(N-(4-chlorophenyl)methylsulfonylamino)-N-methylacetamide), these terms also include various crystalline forms of the compound of Formula I, pharmaceutically acceptable salts;

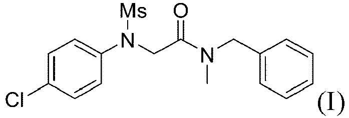

### Ms = methylsulfonyl.

Wherein, the term "pharmaceutically acceptable salts" refers to salts that are formed by the compound of the invention and acids or bases, and suitable for use as a drug. Pharmaceutically acceptable salts include inorganic salts and organic salts. A group of preferred salts are salts formed by the compound of the present invention and the acids. Acids suitable for forming salts include, but are not limited to, inorganic acids such as hydrochloric acid, hydrobromic acid, hydrofluoric acid, sulfuric acid, nitric acid, phosphoric acid and the like; organic acids such as methanoic acid, acetic acid, propionic acid, oxalic acid, malonic acid, succinic acid, fumaric acid, maleic acid, lactic acid, malic acid, tartaric acid, citric acid, bitter acid, methane sulfonic acid, benzenemethanesulfonic acid, benzene sulfonic acid, and the like; and amino acids such as aspartic acid, glutamic acid and the like.

### PHARMACEUTICAL COMPOSITION AND THE ADMINISTRATION THEREOF

The compound of the present invention possesses outstanding activity of inhibiting the TREK-1 ion channel as well as *in vivo* antidepressant ability. Therefore, the compound of the present invention, and the crystal forms thereof, pharmaceutically acceptable inorganic or organic salts thereof, and the pharmaceutical composition containing the compound of the present invention as a main active ingredient can be used for treating, preventing and/or alleviating TREK-1 ion channel-related diseases (e.g. psychiatric disorders) or for treating, preventing and/or alleviating psychiatric disorders such as depression. According to the prior art, the compound of the invention can be used to treat the following diseases: psychiatric disorders such as depression, anxiety disorder, schizophrenia, and/or bipolar disorder.

The pharmaceutical composition of the invention comprises the compound of the present invention (the compound of formula I) or the pharmaceutically acceptable salts thereof in a safe and effective dosage range. The pharmaceutical composition of the invention may further include additional therapeutic agents for depression or antidepressant drugs (such as fluoxetine). The pharmaceutical composition of the invention may further include pharmaceutically acceptable excipients or carriers.

"Safe and effective amount" refers to the amount of compound which is sufficient to significantly improve the condition, and not to generate severe side effects. Generally, the pharmaceutical composition contains 1-2000 mg compound of the invention per dose, preferably, 10-500mg compound of the invention per dose. Preferably, the "one dose" is one capsule or one pill.

"Pharmaceutically acceptable carrier" means one or more compatible solid or liquid fillers, or gel materials which are suitable for human use and should be of sufficient purity and sufficiently low toxicity. "Compatibility" used herein means that the components of the composition can be admixed with the compounds of the invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc.

There is no special restriction on the administration mode of the compound or pharmaceutical compositions of the present invention, and they can be administered by conventional means to the subject in need (e.g., human and non-human mammals). Representative administration mode include (but are not limited to): oral, injectable (e.g., intravenous, intramuscular, or subcutaneous), and inhalation (e.g., aerosol inhalation) administration.

Solid dosage forms for oral administration include capsules, tablets, pills, powders and granules. In these solid dosage forms, the active compounds are mixed with at least one conventional inert excipient (or carrier), such as sodium citrate or dicalcium phosphate, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or the mixtures thereof. In capsules, tablets and pills, the dosage form may also contain buffering agents. The solid dosage form such as tablets, sugar pills, capsules, pills and granules can be prepared by using coating and shell materials, such as enteric coatings and any other materials known in the art. They can contain an opaque agent. The active compounds or compounds in the compositions can be released in a delayed mode in a given portion of the digestive tract (i.e. sustained release preparation). Examples of the embedding components include polymers and waxes. If necessary, the active compound and one or more above excipients can form microcapsules.

Liquid dosage forms for oral administration include pharmaceutically acceptable emulsions, solutions, suspensions, syrups or tinctures. In addition to the active compounds, the liquid dosage form may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

Besides these inert diluents, the composition may also contain additives such as wetting agents, emulsifiers, and suspending agent, sweetener, flavoring agents and perfume.

In addition to the active compounds, the suspension may contain suspending agent, for example, ethoxylated isooctadecanol, polyoxyethylene sorbitol and dehydrated sorbitan esters, microcrystalline cellulose, methanol aluminum and agar, or the combination thereof.

The compositions for injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

The pharmaceutical composition of the present invention can also be made into powders for aerosol inhalation.

Compounds of the present invention can be administrated alone, or in combination with any other pharmaceutically acceptable compounds.

When the pharmaceutical composition is used, a safe and effective amount of drug is administered to a mammal (such as human or a non-human mammal), wherein the safe and effective amount is usually at least about 10 µg/kg body weight and in most cases does not exceed about 50 mg/kg body weight (daily), preferably the dose is about 10 µg/kg body weight to about 20 mg/kg body weight, more preferably, 1 to 20 mg/kg body weight, most preferably 1 to 5 mg/kg body weight. Of course, the specific dose should also depend on various factors, such as the route of administration, the health conditions of patient, which are well within the skills of an experienced physician.

### TREK-1 ion channel

As used herein, the terms "TREK-1 ion channel" and "TREK-1 channel" can be used interchangeably. TREK-1 ion channel (TWIK-Related K⁺ Channel 1) is an important member of the Potassium channel K2P channel (Two Pore-Domain Potassium Channels) family.

### Depression

Depression, also known as depressive disorder, is characterized by significant and persistent depression and is the main type of mood disorder. Clinically, it can be seen that low mood is not commensurate with its situation. Emotional depression can range from gloom to grief, inferiority depression, and even pessimism, and may have suicidal attempts or behaviors; or even the occurrence of stupor. Some cases have obvious anxiety and exercise agitation. In severe cases, psychotic symptoms such as hallucinations and delusions may occur. Each period lasts for at least 2 weeks, or longer or even for several years, and most cases tend to recur, most of which can be relieved, but some may have residual symptoms or become chronic.

The treatment of refractory depression among depressions is more difficult and slower. Clinically, a diagnosis of refractory depression is made after a poor outcome (HAMD reduction rate <20%) after adequate dose and full course of treatment by applying two or more antidepressant drugs with different action mechanisms. Current treatment regimens generally require attempts to administer multiple combinations of drugs at high doses, placing a physical and psychological burden on the patient.

### The main advantages of the present invention include:

(a) The compound CLA096 of the invention has significant antidepressant effect. Especially there is also significant antidepressant effect on refractory depression.
(b) The compound CLA096 of the invention has excellent safety.
(c) The compound CLA096 of the invention can produce the antidepressant effect equivalent to or even significantly superior to the positive drug (fluoxetine) in the body at a lower dose (such as 5mg/kg body weight).
(d) The compound CLA096 of the invention shows significant antidepressant effect in both rats and mice.

The present invention will be further described hereafter in combination with specific examples. It should be understood that these examples are only used to illustrate the present invention and not to limit the scope of the invention. The experimental methods without specific conditions in the following examples generally follow the conventional conditions or the conditions suggested by the manufacturer. Unless otherwise stated, percentages and parts are percentages by weight and parts by weight.

Unless otherwise specified, the test or analysis methods used in each example are as follows:

### Experimental Method of TREK-1 Point Physiological Experiment

### Cell Transfection

Cells were transfected using a cationic liposome transfection method, and the target gene TREK-1 plasmid protein was heterologously expressed in CHO-K1 cells according to the operating standard instructions of Lipo3000 transfection reagent. The ion channel protein was expressed in the cells for 24-48h followed by electrophysiological experiments.

### Electrophysiological experiments and data analysis

Whole-cell membrane clamp recording mode was used for electrophysiological experiments. The transfected cells were placed in a bath containing extracellular fluid, the resistance of the drawn electrode is 3-7 MΩ, the electrode was filled with intracellular fluid, the polished electrode was used to contact the cell membrane, the positive voltage was removed and the negative voltage was gently applied to form a high impedance seal, and then the cell membrane was broken by continuing to apply negative voltage, i.e. whole-cell recording mode was formed. The sampling frequency of the electrophysiological amplifier was 10 kHz, and the data sampling was controlled using a DigiData 1440A conversion interface. The software used for collecting and analyzing whole-cell recording experimental data is Clamfit 10.7, the software used for data analysis and graphing is Origin 8.1, and the software used for statistical analysis is SPSS.

### Animal behavioral methods:

### Open field test (OPT)

Open field test, also known as the open box test, is a method to evaluate the autonomous behavior, exploratory behavior and anxiety-like behavior of experimental animals in a novel environment. The frequency and duration of the normal activities, the fear of the open environment and the exploration of the new environment and other behaviors of the experimental animals in the open box are used to reflect the autonomous behavior and exploratory behavior of the experimental animals in the unfamiliar environment. The results of the open-field experiments were analyzed to evaluate the depression and anxiety-like behaviors of the animals.

Experimental operation: the experimental animals were placed in the behavior room 2 hours before the experiment. The animals were placed in the center of the bottom of a black plastic box (size 100^{∗}100^{∗}45 cm) during the experiment, while video filming was performed. The experiment time was 10 minutes and was conducted in a quiet environment. The bottom and inner walls of the open box were wiped after each experiment to prevent the interference of the remaining information of the last animal on the next experiment. The video was analyzed using the animal spontaneous activity analysis software to obtain information of central area activity time (anxiety-like behavior), total distance of activities (exercise ability), and number of wall climbing (novel environment exploratory behavior) and the like, which can be used to judge the depression or anxiety-like behavior of the animal and degree thereof.

### Forced Swimming Test (FST)

The forced swimming test system is primarily used in antidepressant studies. The experimental animal is placed in a confined environment (e.g., water) wherein the animal struggles desperately to escape but unable to succeed, thus providing an inescapably oppressive environment, and after a period of experimentation, the animal exhibits a typical "immobility state" reflecting a state called "behavior despair state", and a series of parameters in the process of animals in this environment that generate desperate immobility were recorded.

Experimental operation: Mice were individually placed in a cylindrical glass tank 30 cm high and 20 cm in diameter, with a water depth of 15 cm (20 cm for rats), so that the mice are unable to escape from the glass tank and their feet and tails can not touch the bottom of the tank. The water temperature was 23°C-25°C. The mice were filmed for 6 minutes after entering the water. Since most of the mice were very active in the first two minutes, the last 4 minutes of immobility time were calculated (criteria for determining immobility: the mice stopped struggling in the water, immobility and fine limb movements to maintain balance or float). Groups of mice were operated in parallel.

### Novelty-Suppressed Feeding Experiment

Novelty-suppressed feeding experiment (NSF) is a conflict between feeding occurred in a novel environment in a state of starvation after fasting and fear of novel environment. Long-term administration of antidepressant drugs can reduce the conflict response ability of animals, which is manifested by shortened incubation period of the start of feeding. This model is currently widely used in the evaluation of antidepressant and anti-anxiety efficacy.

The experimental equipment was a 35cm*35cm*30cm (L*W*H) open box, which was overspread with substrate, and a white filter paper with a diameter of 5cm was placed in the open box, and 2 pieces of food were placed in the middle of the filter paper. The animals were fasted for 12 h before the administration of the drug.

Two hours before the behavioral test, the animal were moved into the functional room for 2 h to acclimatize. The drug intervention was given according to the above dosing protocol. Novelty-suppressed feeding experiments were performed at 1h and 24h after drug administration, respectively, and were performed as follows. The animal were placed from the edge of the experimental open box, and the animal were regarded as successful feeding if their mouths touched the food within 10 min, and then the animal were removed immediately and the time of successful feeding (i.e. Novelty latency) was calculated. The animal were then placed in the normal feeding cage and the time from placement to feeding (i.e. Habituation latency) was also calculated. If the animal did not successfully obtain food within 10 min, the animal were forcibly removed and similarly placed in the normal cage, and the habituation latency thereof was also calculated. The whole experiment was recorded by camera, and the data were processed and analyzed offline.

### Example 1 Preparation of CLA096

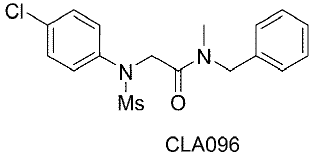

### Synthetic route:

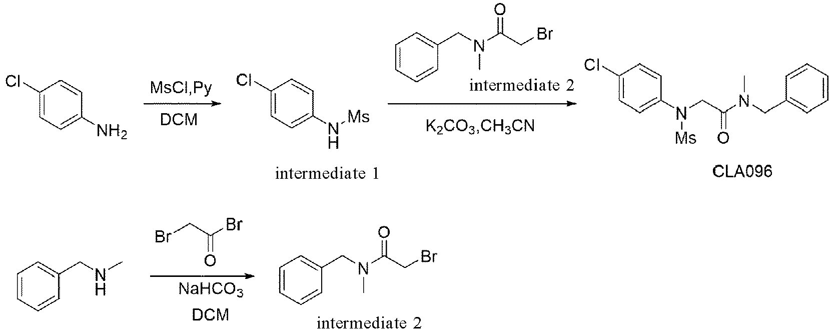

**STEP 1:** *P*-chloroaniline (150.00 g, 1.18 mol) was dissolved in dichloromethane (1.25 L) and pyridine (102.83 g, 1.30 mol) was added at room temperature at once. The temperature of the reaction system remained unchanged. The reaction was replaced with nitrogen for three times and cooled to 0~5°C in an ice-water bath, and methyl sulfonyl chloride (148.67 g, 1.30 mol) was added slowly dropwise. After dripping, the reaction was warmed to room temperature and stirred overnight, TLC (PE: EA=3:1) shows that the reaction of raw material *p*-chloroaniline was complete. The reaction solution was quenched by pouring into water (2.0 L) and solids were precipitated, filtered and the filter cake was washed with water and dried to give intermediate 1 (236.80 g, 98% yield) as light pink solid.
HPLC: 99.73% purity, 220 nm; ¹H NMR: (400 MHz, DMSO-d6) δ9.87 (s, 1H), 7.43-7.36 (m, 2H), 7.24-7.17 (m, 2H), 2.99 (s, 3H).

**STEP 2:** Sodium bicarbonate (156.10 g, 1.86 mol) was dispersed into dichloromethane (0.5 L), bromoacetyl bromide (274.83 g, 1.36 mol) in dichloromethane (0.5 L) was added dropwise through a dropping funnel. After addition, the reaction was replaced with nitrogen for three times and cooled to 0 to 5 degrees in an ice water bath. N-methylbenzylamine (150.00 g, 1.24 mol) in dichloromethane (0.5 L) was added slowly dropwise through the dropping funnel, and after dropping, the solution was warmed to room temperature and stirred for 1 h. The HPLC showed that there was still a part of N-methylbenzylamine remaining. The reaction was cooled to 0 to 5 degrees in an ice-water bath, supplemental bromoacetyl bromide (75.00 g, 0.37 mol) was added and the mixture was stirred overnight at room temperature, and HPLC showed that a small amount of N-methylbenzylamine was still remaining. The reaction solution was quenched by pouring into ice water (1.5 L). The mixture was partitioned, extracted with dichloromethane. The organic phase was combined, part of the organic phase was concentrated, dried with anhydrous sodium sulfate, filtered, concentrated, and the crude product was purified by silica gel column chromatography (PE:EA=10: 1 to 4:1) to obtain intermediate 2 (200 g, 67% yield) as colorless oily product. HPLC: (98.75% purity, 220 nm)

**STEP 3:** Intermediate 1 (232.0 g, 1.13 mol), Intermediate 2 (309.9 g, 1.28 mol) and potassium carbonate (235.0 g, 1.70 mol) were dispersed in acetonitrile (2.5 L), heated and stirred at reflux for 2 h. HPLC showed that the reaction of the raw materials was complete. The reaction solution was cooled to room temperature, quenched with water (1.5 L), partitioned, extracted with ethyl acetate. The organic phases were combined, part of the organic phase was concentrated, washed with water, dried with anhydrous sodium sulfate, filtered and concentrated to obtain crude product as pale yellow oil, which was purified by silica gel column chromatography (petroleum ether: ethyl acetate=10: 1 to 4:1) to give an off-white solid CLA096 (245.6 g, 59% yield). LCMS: m/z=367.1 [M+H]⁺, HPLC: (99.33% purity, 220 nm), ¹H NMR (400 MHz, DMSO-*d*₆) δ7.53-7.44 (m, 4H), 7.38-7.23 (m, 3H), 7.19-7.10 (m, 2H), 4.70-4.67 (m, 2H), 4.56-4.49 (m, 2H), 3.14 (s, 3H), 2.91-2.81 (m, 3H).

### Example 2 Effects of CLA096 on TREK-1 Channels

Specific implementation:
Test drug: CLA096
Test drug configuration

CLA096: Three doses were set up in this example. The drug was firstly dissolved in DMSO and then diluted with tri-distilled water to high, medium and low doses: 5 µM, 10 µM and 20 µM, respectively.

The experiment was performed using a cell membrane clamp method to record the current of TREK-1 ion channel in whole cells. Protocol A0 was chosen to record the currents of cells at -80mV, -60mV, -40mV, -20mV, 0mV, 20mV, 40mV, 60mV and 80mV, and Protocol A0 was repeated with different concentrations such as 5µM, 10µM and 20µM of CLA096 being perfused, respectively. After administration, whether it can be eluted was tested.

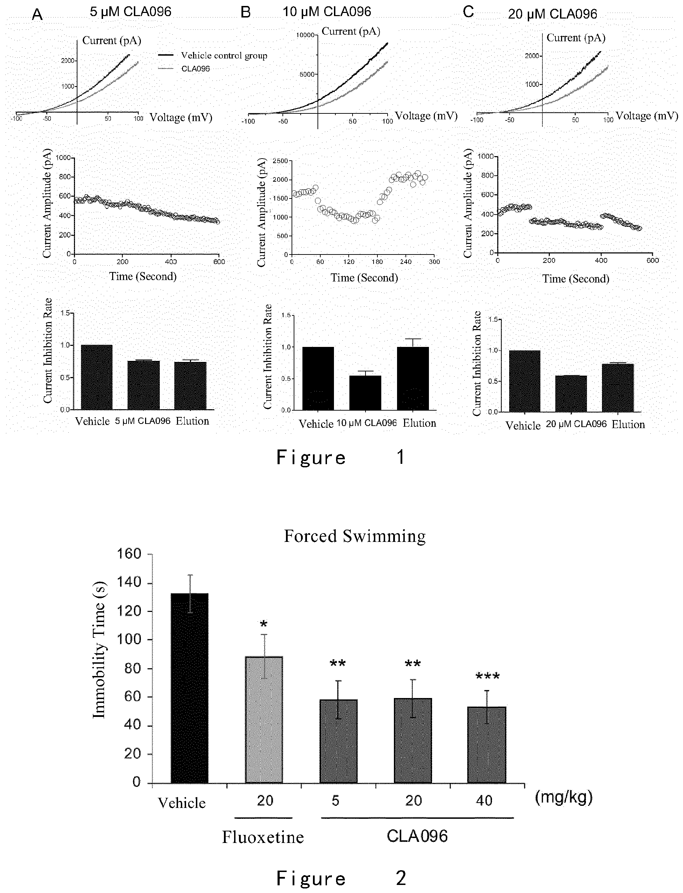

The results are shown in Figure 1, CLA096 is able to inhibit the open current of TREK-1 channel, wherein the inhibition rates of 5 µM, 10 µM and 20 µM CLA096 are 0.75 ± 0.02, 0.55 ± 0.05 and 0.59 ± 0.01, respectively, indicating the high inhibitory activity of CLA096 on TREK-1 channel.

### Example 3 Antidepressant effect of compound CLA096 in C57 mice

C57 mice were selected in the present invention, and two kinds of behavioral pharmacodynamic experiments of antidepressants were conducted using different doses of CLA096 by intraperitoneal administration. The compound CLA096 was found to have a significant antidepressant effect, which was superior to that of the positive control drug fluoxetine, and therefore can be used as an application for the preparation of drugs for the treatment of depression.

### The experimental procedure and specific results are as follows.

Test drug:
CLA096, Fluoxetine (FLX)
Test drug configuration

CLA096: Three doses were set up in this example, the low, medium and high doses were 5 mg/kg, 20 mg/kg and 40 mg/kg, respectively. The compound was dissolved in 0.5% Tween-40 and sonicated, and should be used right after it was ready.

Fluoxetine (FLX): FLX with a dose of 10 mg/kg; dissolved in double distilled water, and should be used right after it was ready.

Vehicle control group (Veh): 0.5% Tween-40.

Data analysis:
All data analysis were completed using spss 22 (for mac) data processing software. One-way analysis of variance was used and results were calibrated using LSD method for multiple comparisons.

Animal grouping
60 of 8-week-old C57 male mice were randomly divided into five groups, and each group was shown in the following table:

| Group | Number of Animals (mouse) | Administration mode | Injection Dose (mg/kg) | Injection Volume (ml/kg) |
|---|---|---|---|---|
| Vehicle control group | 12 | intraperitoneal injection | 0.5% Tween-40 | 10 |
| Fluoxetine group | 12 | intraperitoneal injection | 20 | 10 |
| CLA096 low dose group | 12 | intraperitoneal injection | 5 | 10 |
| CLA096 medium dose group | 12 | intraperitoneal injection | 20 | 10 |
| CLA096 high dose group | 12 | intraperitoneal injection | 40 | 10 |

Experimental results
1. Forced swimming experiment in mice Figure 2 shows the immobility time (s) of mice in forced swimming test, the results shows that fluoxetine significantly reduces the immobility time of animals in water, CLA096 significantly reduces the immobility time of animals at all doses, and the effect of CLA096 is significantly better than fluoxetine. The immobility time of forced swimming reflects the desperate behavior of animals in an inescapable environment, which is also a common depressive symptom, and it can be seen that CLA096 has a significant antidepressant effect. (Data were analyzed using One-way ANOVA with LSD *post-hoc* tests: ^{∗}p<0.05, ^{∗∗}p<0.01, ^{∗∗∗}p<0.001, compared with vehicle control group).
2. Novelty-Suppressed Feeding Inhibition Experiment

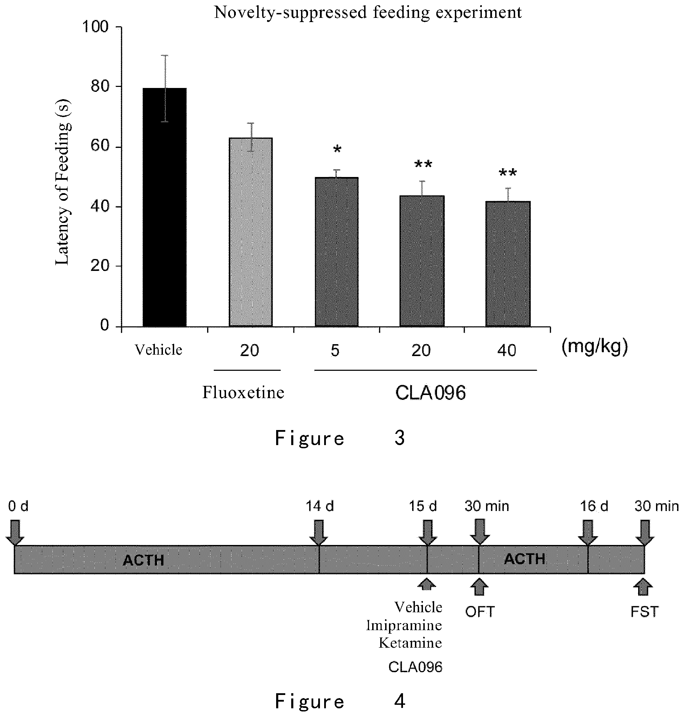

Figure 3 shows the determination results of the latency (time, s) of the compound on feeding of fasted mice. The value of latency reflects the motivation of mice in a physiological sense. It is one of the clinical symptoms of depression and can also be used to describe anxiety symptoms. In general, the longer the latency period, the severer the depressive symptoms. All different doses of CLA096 significantly reduced the latency value and were significantly better than fluoxetine (data were analyzed using One-way ANOVA with LSD *post-hoc* tests: ^{∗}p<0.05, ^{∗∗}p<0.01, compared with vehicle control group).

Based on the results of the above two behavioral pharmacodynamic experiments of antidepressant, it is shown that CLA096 has a significant antidepressant effect, and the antidepressant effects of CLA096 at all three doses of high, medium and low are better than that of the positive control drug fluoxetine. It can be seen that the CLA096 of the invention is able to bring a significant antidepressant-like behavior at low doses.

### Example 4 Antidepressant efficacy study of CLA096 in a rat model of refractory depression

Modeling method:
An animal model of refractory depression was induced in SD rats by continuous intraperitoneal injection of adrenocorticotropic hormone (ACTH) for 3 weeks. This model can block the effects of tricyclic antidepressants and some SSRIs antidepressants, but can not block the efficacy of ketamine and electroconvulsive therapy (ETC). Since ketamine and ETC have specific efficacy in refractory depression, this animal model can be used to preliminary evaluate whether the drug have efficacy in refractory depression.

The experimental procedure is generally shown in Figure 4, and the specific steps are as follows:
1. SD rats in the model group (ACTG) were continuously injected with ACTH for 3 weeks (once/day), and the test drug was injected intraperitoneally 24 h after the last administration of ACTH. The rats in the control group (CON) were continuously injected intraperitoneally with 0.9% saline (once/day) and similarly the test drug was injected intraperitoneally 24 h after the last administration of saline.
2. After 30 min of single dose administration of the test drug, the open-field experiment was performed.
3. After the open-field experiment, ACTH was continued to be injected, and after 24 h, the above test drug was injected intraperitoneally and forced swimming was tested 30 min after the administration.

### Experimental groupings:

### Each groups are shown in the following table:

| Group | Injection Dose (mg/kg) | Injection Volume (ml/kg) | Administration mode | Behavioral test |
|---|---|---|---|---|
| CON+Veh | Vehicle | 10 | intraperitoneal injection | Open field is tested 30 min after administration, and forced swimming is tested 30 min after administration the next day. |
| CON+Imipramine | 20 | 10 | intraperitoneal injection | |
| CON+Ketamine | 20 | 10 | intraperitoneal injection | |
| CON+CLA096 | 20 | 10 | intraperitoneal injection | |
| ACTH+Veh | Vehicle | 10 | intraperitoneal injection | |
| ACTH +Imipramine | 20 | 10 | intraperitoneal injection | |
| ACTH +Ketamine | 20 | 10 | intraperitoneal injection | |
| ACTH +CLA096 | 20 | 10 | intraperitoneal injection | |

### 1. Results of forced swimming in rats

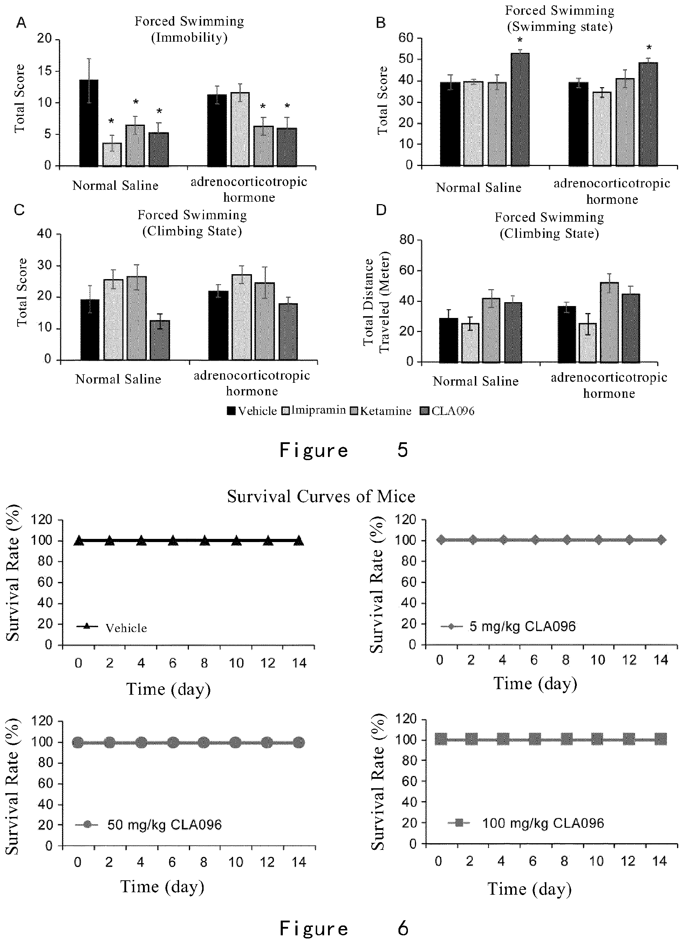

The results of forced swimming in rats showed (Figure 5. A-C) that CLA096 and the positive drugs Imipramine and ketamine significantly reduced the immobility time of forced swimming in rats in the control (CON) group (Figure 5A), and CLA096 significantly increased the swimming time of rats (Figure 5B), indicating that the antidepressant efficacy of CLA096 was significant with acute administration. The antidepressant effect of the tricyclic antidepressant Imipramine was blocked after rats were modeled with ACTH (ACTH group rats), while the antidepressant effect of Ketamine and CLA096 remained significant, indicating that CLA096 has effect on refractory depression (data were analyzed using One-way ANOVA with LSD *post-hoc* tests: ^{∗}*p*<0.05, compared with vehicle control group).

### 2. Results of the rat open-field experiment

The rat open field results (Figure 5D) showed that although CLA096 and Ketamine had an increasing trend in the total movement distance of normal animals and ACTH model animals, there was no significant difference, indicating that CLA096 had no significant effect on the movement ability of rats.

### Example 5 14-day subacute toxicity test of CLA096

Specific implementation:
Test drug: CLA096
Test drug configuration: CLA096: Three doses were set up: 5 mg/kg, 50 mg/kg and 100 mg/kg, respectively.

5 mg/kg: taking 8.2 µl of stock solution, adding 155.3 µl of DMSO, then adding 327 µl of Tween-80, mixing thoroughly, and finally adding tri-distilled water to a required volume of 3 ml, and tri-distilled water is added slowly in drops to prevent the precipitation of drugs.

50 mg/kg: taking 81.8 µl of stock solution, adding 81.7 µl of DMSO, then adding 327 µl of Tween-80, mixing thoroughly, and finally adding tri-distilled water to a required volume of 3 ml, and tri-distilled water is added slowly in drops to prevent the precipitation of drugs.

100 mg/kg: taking 163.5 µl of stock solution, then adding 327 µl of Tween-80, and finally adding tri-distilled water to a required volume of 3 ml, and tri-distilled water is added slowly in drops to prevent the precipitation of drugs.

Vehicle control group: taking 163.5 µl DMSO, then adding 327 µl Tween-80, and finally adding tri-distilled water to a required volume of 3 ml.

Animal grouping: 20 male mice and 20 female mice were randomly divided into 4 groups, specific groups are as shown in the following table:

| Groups | Administration Dose (mg/kg) | Administration Volume (ml/kg) | Administration frequency (times / day) | Administration Times (times) | Number of Animals (mouse) | Administration mode |
|---|---|---|---|---|---|---|
| Vehicle control | Vehicle of the test compound being used as control. | 10 | 1 | 14 | 5 male mice and 5 female mice | intraperitoneal |
| Low dose CLA096 | 5 | 10 | 1 | 14 | 5 male mice and 5 female mice | intraperitoneal |
| medium dose CLA096 | 50 | 10 | 1 | 14 | 5 male mice and 5 female mice | intraperitoneal |
| high dose CLA096 | 100 | 10 | 1 | 14 | 5 male mice and 5 female mice | intraperitoneal |

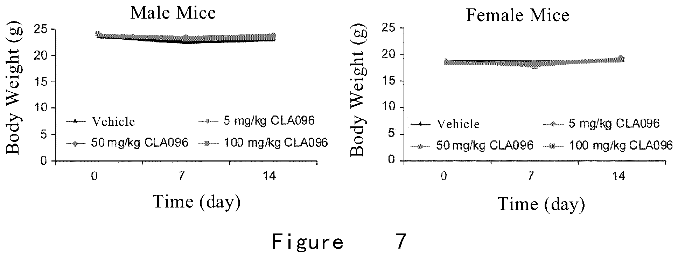

It can be seen from the survival curves of mice shown in Figure 6 that the low, medium and high dose groups of CLA096 were not lethal to the mice. Moreover, no significant changes in body weight were observed from any of the mouse groups of both gender (see Figure 7).

Based on the above behavioral pharmacodynamic experimental results, it is indicated that CLA096 possesses significant and excellent antidepressant effect and a good safety profile.

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

1. A use of a compound of formula I or a pharmaceutically acceptable salt thereof in (i) the preparation of a pharmaceutical composition or a preparation for preventing and/or treating a TREK-1 ion channel-related disease, (ii) the preparation of a pharmaceutical composition or a preparation for preventing and/or treating a psychiatric disorder and/or (iii) the preparation of a TREK-1 ion channel inhibitor.

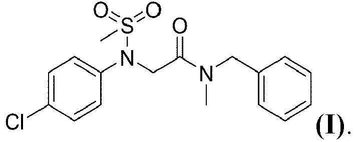

2. The use according to claim 1, wherein the TREK-1 ion channel-related disease includes a psychiatric disorder.

3. The use according to claim 1 or 2, wherein the psychiatric disorder includes depression, anxiety disorder, schizophrenia, bipolar disorder, or a combination thereof.

4. The use according to claim 3, wherein the depression includes major depressive disorder and/or refractory depression.

5. The use according to claim 1, wherein the administration dosage of the compound of formula I is 0.1-50 mg/kg body weight, preferably 1-5 mg/kg body weight.

6. The use according to claim 1, wherein the pharmaceutical composition contains 0.001 to 99 wt% the compound of formula I or the pharmaceutically acceptable salt thereof, based on the total weight of the pharmaceutical composition.

7. The use according to claim 1, wherein the dosage form of the pharmaceutical composition includes an oral dosage form, an injection dosage form, and/or an inhalation dosage form.

8. A pharmaceutical composition, wherein the pharmaceutical composition comprises a compound of Formula I or a pharmaceutically acceptable salt thereof.

9. The pharmaceutical composition according to claim 8, wherein the pharmaceutical composition further includes one or more additional active ingredients selected from the group consisting of
(i) a TREK-1 ion channel inhibitor;
(ii) a drug for preventing or treating a psychiatric disorder.

10. The pharmaceutical composition according to claim 8, wherein the pharmaceutical composition further contains pharmaceutically acceptable carriers.

11. A method for preventing and/or treating a TREK-1 ion channel-related disease, comprising a step of administration of a compound of Formula I or a pharmaceutically acceptable salt thereof to a subject in need thereof; wherein the compound of formula I is as defined in claim 1.

12. The method according to claim 11, wherein the TREK-1 ion channel-related disease is a psychiatric disorder.

13. The method according to claim 11, wherein the TREK-1 ion channel-related disease includes depression, anxiety disorder, schizophrenia, bipolar disorder, or a combination thereof.

14. A kit, wherein the kit comprising:
(i) a drug or a preparation containing a compound of formula I or a pharmaceutically acceptable salt thereof as an active ingredient; and
(ii) an instructions.
